# EUROPEAN PATENT SPECIFICATION

(11) **EP 1 071 362 B1**
(45) Date of publication and mention of the grant of the patent: **14.12.2005**
(21) Application number: 99914935.4
(22) Date of filing: 18.03.1999
(51) Int. Cl.: B05D 7/22, A61M 5/32, A61B 5/14, A61L 33/00

(54) **ANTICOAGULANT INTERNALLY COATED NEEDLE AND METHOD OF MANUFACTURING SAME**
INNENSEITIG MIT ANTIKOAGULANT BESCHICHTETE NADEL UND ZUGEHÖRIGES HERSTELLUNGSVERFAHREN
AIGUILLE INTERIEUREMENT COUVERTE D'UN ANTICOAGULANT ET SA METHODE DE FABRICATION

(30) Priority: 19.03.1998 US 44149
(43) Date of publication of application: 31.01.2001
(73) Proprietor: PORTEX, INC., Keene, New Hampshire 03431-5911 (US)
(72) Inventor: DUCHAMP, Jacky, San Francisco, CA 94114 (US)
(74) Representative: Gill, Ian Stephen
(86) International application number: PCT/US1999/005888
(87) International publication number: WO 1999/047037

(56) References cited:
- EP-A- 0 109 970
- US-A- 4 133 304
- US-A- 4 327 722
- US-A- 4 409 990
- US-A- 4 424 817
- US-A- 4 572 210
- US-A- 4 653 511
- US-A- 4 808 449
- US-A- 5 232 455
- US-A- 5 584 816

## Description

The present invention relates to blood drawing devices, and more particularly a blood drawing device that has a needle internally coated with an anticoagulant so that when the needle is used to puncture the vein of a patient, blood that flows through the needle is clot free.

In the medical field, in addition to being known as one of the more important medications, heparin is used to inhibit coagulation of blood drawn for blood gas analysis and other laboratory tests. In conventional commercial blood drawing tubes or syringes, the final concentration of heparin is known to be about 3-200 units/ml. This concentration of heparin is by far and away sufficient to anticoagulate blood drawn from a patient.

Based on a study carried out by the departments of pathology, biochemistry and pediatrics of the University of Colorado Health Sciences Center in Denver, Colorado, the concentration of heparin (bovine sodium heparin) necessary to inhibit clotting was found to be 0.6 and 0.75 units/ml of plasma and 0.4 and 0.6 units/ml of whole blood. No visual clots or instrumentation interference due to micro-clots were observed in whole blood anticoagulated with a minimum of 1.0 unit/ml of heparin after 20 minutes at room temperature or 75 minutes at 4°C. At 4°C, the minimum heparin concentration to inhibit whole blood from clotting for over 4 hours was 0.75 unit/ml.

However, since most manufacturers of blood drawing devices use some form of dry heparin (vaporized or lyophilized) in the body of the device, occasional premature clots particularly inside the inner surface of the cannula, would occur. So, too, because of the different degrees of roughness of the inner surface of the cannula and the uniqueness of the clotting factors which vary from individual to individual, it is not uncommon for micro-clots to form in the blood prior to the blood reaching the bulk of the heparin in the blood storage device. The phenomenon does not occur in blood drawing devices that contain a form of liquid heparin. This is due to the fact that as the liquid heparin is expelled prior to use (by the user pushing the plunger of the syringe in the direction of the needle), the inner surface of the cannula is indirectly coated, thereby enabling the blood to be in contact with the anticoagulant as soon as it is drawn from the patient. But, a problem does arise with the use of such blood drawing device because of the dilution effect that liquid heparin has on the blood drawn, as the accuracy of the measurement of the various components in the blood drawn from the patient is affected by the blood having been diluted with the liquid heparin.

In view of the disadvantages of the conventional types of blood drawing devices coated with dry heparin and liquid heparin, there is therefore a need for a blood drawing device coated with an anticoagulant that will not cause micro0clots or be affected by any dilution effect of the blood withdrawn from the patient. Putting it differently, there is a need for a blood drawing device that would not adversely influence the analysis of pH/blood gas of drawn blood.

US-A-4808449 discloses a method of coating the interior surface of containers such as syringes with biological agents by atomising the agent inside the container and relieves the pressure within the container to allow the agent to auto-freeze dry on the surface of the container.

EP 0109970 discloses a syringe having a needle which has prepackaged liquid heparin in a space between a stopper and the neck of the syringe, so that the hollow shank of the needle may be filled with the anticoagulant simultaneously with the evacuation of possible air from the syringe chamber, prior to use of the needle on a patient.

According to one aspect of the present invention there is provided a needle assembly adaptable to be used with a syringe, comprising: a hub; and a needle extending from said hub; characterised by: a liquid anticoagulant dispensed to and dried onto the interior surface of said needle to form a layer of crystallyzed anticoagulant onto the interior surface of said needle prior to said needle being used to puncture the vein of a patient, whereby said crystallisation coat of anticoagulant prevents blood drawn from the patient from clotting in said needle.

The present invention further provides a method of manufacturing a nonclottable device for withdrawing clottable fluid from a patient comprising the steps of: a) fixing a needle to a hub; and (b) atomising a liquid anticoagulant into said needle form a layer of crystallized anticoagulant to the interior surface of said needle onto the interior surface of said needle prior to said needle being used to puncture the vein of a patient, whereby said layer of anticoagulant prevents blood drawn from the patient from clotting in said needle.
The present invention still further provides a needle and syringe assembly comprising a needle assembly according to the invention, and a filter adaptable to let air pass but block the passage of liquid fitted to said syringe to allow any air bubbles in said blood collected from the patient and being stored in said syringe to pass through said filter.

To overcome the disadvantages of the prior art blood drawing devices, the present invention blood drawing device has the interior or inner surface of its needle dispensed with a liquid anticoagulant, preferably by an ultrasonic atomisation process. The thus atomized needle is then dried, either by air or by heat, so that the interior surface thereof is coated with a layer of anticoagulant at a concentration that comports with the above-noted study done by the University of Colorado Health Sciences Center. The thus anticoagulated needle can be used with a syringe, or container, that is fitted with an air bubble removable filter such as the FILTER-PRO™ of the assignee. The syringe can also have attached thereto the needle protection sheath (NEEDLE-PRO™) of the assignee.

An objective of the present invention blood drawing device is to prevent any dilution effect that may occur when an anticoagulant is used with a blood withdrawing device.

Another objective of the present invention is to provide a blood drawing device having a needle that does not require to be coated by any liquid heparin just prior to its being used.

Yet another objective of the present invention is the provision of a blood drawing device that has a stable, non-reactive coating of an anticoagulant already formed in the inner surface of its needle, so that the device has an extended period of shelf store life and can be used right from the package.

The above-mentioned objectives and advantages of the present invention will become more apparent and the invention itself will be best understood by reference to the following description of an embodiment of the invention taken in conjunction with the accompanying drawings, wherein
Fig. 1 is an illustration of a needle of the present invention and its being atomized with a liquid anticoagulant; and
Fig. 2 is a side view of a blood drawing device of the present invention which includes a semi-cut away cross-sectional view of a syringe mated to the hub of a needle assembly of the present invention.

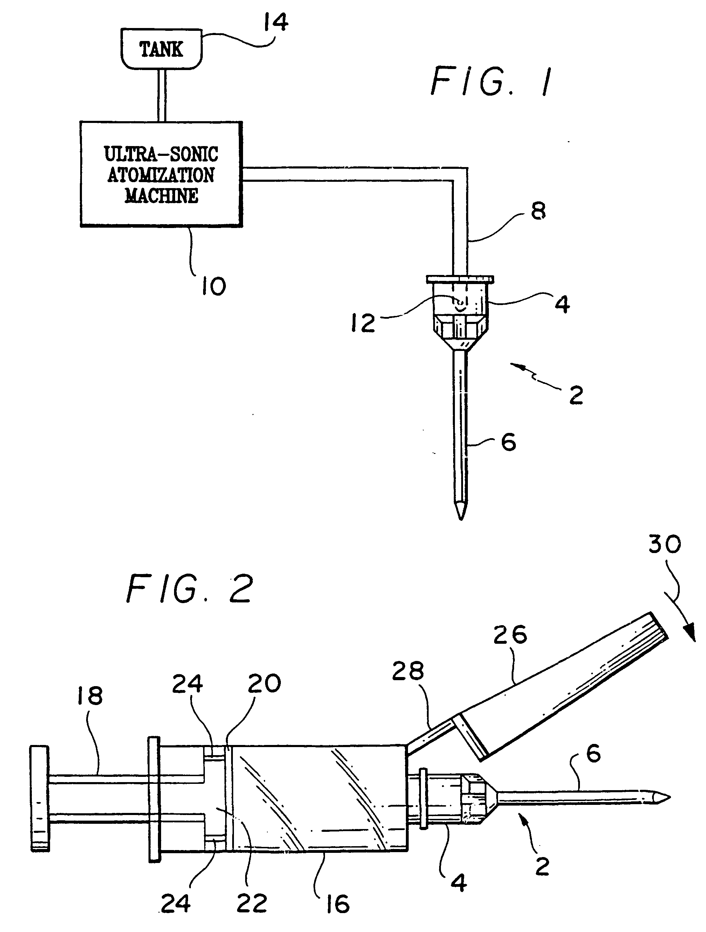

With reference to Fig. 1, the present invention needle assembly 2 is shown to include a hub 4 and a needle 6 made of stainless steel. In the preferred embodiment, the stainless steel used for needle 6 is the 304 type. However, other types of known stainless steel are also suitable for use.

Hub 4 of needle assembly 2 may be a color-coated translucent hub. In the preferred embodiment, needle 6 may be of various sizes including, for example, Regular (A), Intermediate (IN), and all short (B) type needles.

As further shown in Fig. 1, the inner surface of needle assembly 2 is atomized by a liquid anticoagulant, which may be an aqueous solution of heparin provided by a needle tube 8 output from an ultra-sonic atomization machine 10. In particular, the liquid anticoagulant is dispensed or sprayed from at least one hole, such as 12, provided at the tip of needle tube 8. Ultra-sonic atomization machine 10 in essence is comprised of two components, an atomizer machine made by the Ivek Corporation of Massachusetts having model number 2325 and a pump made by the Digispense Corporation having model number 1586. Note that the liquid anticoagulant being provided to ultra-sonic atomization machine 10 is stored in a pressure tank 14. The amount of liquid anticoagulant being pumped from ultra-sonic atomization machine 10 can be regulated, both in terms of its volume and concentration, so that the appropriate amount of anticoagulant is atomized to at least one portion of the interior surface of hub 4 and the interior surface of needle 6.

In the preferred embodiment, note that the liquid anticoagulant is an aqueous solution of heparin which is made with lithium heparin powder mixed with ionized water. However, other types of known anticoagulant substances and associated solvent solutions are also suitable for use with the present invention. These include ammonium heparin, zinc heparin, calcium heparin, low molecular weight heparin, sodium heparin, benzalkonium heparin, dermatan heparin, heparin fragments, and heparin peptidoglycan associated (or not associated) under a calcium neutralized form and/or an electrolyte balanced form with de-ionized water, methylene chloride, isopropanol, and 50:50 (v/v) toluene-petroleum ether. Further note that any combinations of the different elements and substances mentioned hereinabove are also suitable for use in the present invention as the liquid anticoagulant.

As noted above, the ultra-sonic atomization machine 10 determines the volume and/or the concentration of the liquid anticoagulant to be atomized to needle assembly 2. The volume of the anticoagulant dispensed in each needle assembly must take into account the length of the needle, and the final anticoagulant concentration that is desired, as for example between 0-200 units/ml per needle. The atomization process is conventional and need not further be discussed. However, note that machine 10 dispenses the liquid anticoagulant by ultrasonic atomization, so that the liquid anticoagulant being coated onto the inner surfaces of hub 4 and needle 6 is provided as an even patterned spray, with sufficiently small droplet size and uniformity. Further note that in addition to ultra-sonic atomization, other types of atomizers may also be used. These include pressure nozzles (hydraulic), two-fluid nozzles (pneumatic), and rotary devices such as spinning cups, disks, or vaned wheels.

The needle assembly 2 of the instant invention, to be useful, also needs to have its anticoagulant coat to be in a stable state. This means that the coat of anticoagulant in the interior portion of the needle assembly 2 has to be dry, so that all liquid in the anticoagulant has evaporated and that the residual anticoagulant coated onto the interior portions of the needle assembly is crystallized. This drying process can be effected in an oven or air dried. Other types of known drying process are also suitable for the instant invention. Some of these conventional processes include using, for example, continuous and batch dryers, infrared or radiant heat dryers, dielectric heat dryers, and continuous and batch indirect dryers. By providing a dry coat of anticoagulant inside the needle assembly, no corrosion due to contact between any liquid (or condensation) to the metal surface of the cannula would occur.

An example of the process of atomization of liquid anticoagulant to the interior portion of the needle assembly 2 is given herein. For the exemplar process, 5 ul of lithium heparin solution was dispensed inside hub 4 of three categories of needles (22x25mmg, 23x25mmg, and 25x15mmg) (22x1"g, 23x1"g, and 25x5/8"g) (g=gauge). The optimum concentration for the heparin solution was found to be 15 g/l (or equivalent to 2595 units). After the different needles were dried for example by air drying overnight, 4-5 USP units of heparin per needle per ml was recovered, either by an atomic absorption lithium ion study, or by USP potency assay, both processes being conventional and need not be discussed further herein.

With reference to Fig. 2, the present invention needle assembly 2 is shown to mate by means of its hub 4, to a syringe 16. Syringe 16 acts as a container to which blood withdrawn from a patient per needle 6 is collected. As discussed above, by coating the interior surface of needle 6, and at least a portion of the interior surface of hub 4, clots, micro-clots or otherwise, are prevented from occurring while the blood transits from the patient through needle 6 and hub 4, to syringe 16.

To ensure that air bubbles that may be in the blood are removed from syringe 16, the tip of plunger 18 of syringe 16 is fitted with either a hydrophilic or hydrophobic filter 20. Such filter may be the FILTER PRO™ used by the assignee company of the instant invention. Moreover, instead of being at the end of plunger 18, filter 20 may be fitted within a space at end portion 22 of plunger, so that its end face does not come into direct contact with the withdrawn blood. To enable air bubbles to escape from syringe 16, a number of passages, such as 24, may be provided to the end portion of syringe 18, so that as the syringe is filled with blood, any air bubbles therein are forced out into the environment via air passages 24. This is of course due to the fact that air filter 20 has the characteristic of allowing air to pass but blocking the passage of liquids.

Another attribute of the blood drawing device shown in Fig. 2 is a needle protection sheath 26 that is pivotally attached or connected to syringe 16 by means of a living hinge 28. The construction and operation of protection sheath 26 is given in U.S. patent 5,232,455. In brief, after usage, the contaminated needle 6 is prevented from being exposed to the environment by the user pivoting needle protection sheath 26 in the direction of arrow 30 so that sheath 26 comes into alignment with needle 6. A hook, not shown, or other means in sheath 26 (or coacting with hub 4 of needle assembly 2) fixedly retains needle 6 relative to sheath 26 once sheath 26 has been pivoted into alignment with needle 6.

Inasmuch as the present invention is subject to many variations, modifications and changes in detail, it is intended that all matter described throughout this specification and shown in the accompanying drawings be interpreted as illustrative only and not in a limiting sense.

## Claims

1. A needle assembly adaptable to be used with a syringe (16), comprising:
a hub (4); and
a needle (6) extending from said hub;
**characterised by**: a liquid anticoagulant dispensed to and dried onto the interior surface of said needle (6) to form a layer of crystallized anticoagulant thereon onto the interior surface of said needle (6) prior to said needle (6) being used to puncture the vein of a patient, whereby said layer of anticoagulant prevents blood drawn from the patient from clotting in said needle (6).

2. A needle assembly of claim 1 wherein said anticoagulant comprises a heparin substance.

3. A needle assembly according to claim 2, herein said heparin substance comprises lithium heparin powder mixed with de-ionised water.

4. A needle assembly according to claim 2 or claim 3, wherein said heparin substance comprises any of ammonium heparin, zinc heparin, calcium heparin, low molecular weight heparin, sodium heparin, benzalkonium heparin, dermatome heparin, heparin fragments, and various forms of heparin peptidoglycan.

5. A needle assembly according to any of the preceding claims, further comprising:
a sheath (26) flexibly coupled, in use, to a syringe (16) for fixedly enveloping said needle (6) after said needle (6) has been used.

6. A needle assembly according to any one of the preceding claims, wherein the liquid anticoagulant is dispensed to and dried onto at least one portion of the interior surface of said hub (4) to form a dry anticoagulant layer thereon.

7. A needle and syringe assembly comprising a needle assembly according to any of the preceding claims, and
a filter (20) adaptable to let air pass but block the passage of liquid fitted to said syringe (16) to allow any air bubbles in said blood collected from the patient and being stored in said syringe (16) to pass through said filter (20).

8. A method of manufacturing a nonclottable device for withdrawing clottable fluid from a patient comprising the steps of:
a) fixing a needle (6) to a hub (4); and
b) atomising a liquid anticoagulant into said needle (6) to form a layer of crystallized anticoagulant onto the interior surface of said needle prior to said needle being used to puncture the vein of a patient, whereby said layer of anticoagulant prevents blood drawn from the patient from clotting in said needle.

9. A method according to claim 8, wherein said step b) further comprises the step of atomising said anticoagulant to at least one portion of the interior surface of said hub (4).

10. A method of according to claim 8 or claim 9, further comprising the steps of:
mating said needle (6) to a syringe (16); and
flexibly coupling a sheath (26) to said syringe (16), said sheath (26) being pivotable to fixedly envelop said needle (6).

11. A method according to any of claims 8 to 10, further comprising the steps of:
mating said hub (4) to a syringe (16); and
fitting a filter (20) adaptable to allow air to pass but block the passage of fluid to said syringe to allow any air bubbles in said fluid withdrawn from a patient to pass therethrough.

12. A method according to any of claims 8 to 10, further comprising the step of:
drying said anticoagulant after it has been atomized to the interior surface of said needle (6).

13. A method according to any of claims 8 to 11, wherein said anticoagulant comprises a heparin substance, and wherein said step b) further comprises the step of:
atomising said heparin substance to the interior surface of said needle (6) via ultra-sonic atomisation.

14. A method according to any of claims 8 to 12, further comprising the step of:
dispensing said anticoagulant to atomise the interior surface of said needle (6) in a volume and/or concentration dependent on the length of said needle (6).

15. A method according to any of claims 8 to 13, wherein said step b) further comprises atomising the interior surface of said needle (6) with a lithium heparin power mixed with de-ionised water.

16. A method according to any of claims 8 to 14, wherein said step b) further comprises atomising the interior surface of said needle (6) with any of ammonium heparin, zinc heparin, calcium heparin, low molecular weight heparin, sodium heparin, benzelkonium heparin, dermatone heparin, heparin fragments, and various forms of heparin peptidoglycan

17. A method according to any of claims 8 to 15, comprising the further step of varying the volume and/or concentration of said anticoagulant atomised to the interior surface of said needle (6) depending on at least the length of said needle (6) and the desired anticoagulant concentration in said needle (6).

18. A method according to any of claims 9 to 16, wherein said anticoagulant is atomised to the interior surface of said needle (6) via ultra-sonic atomisation.

19. A method according to any of claims 8 to 17, comprising the further step of atomising anticoagulant onto at least one portion of the interior surface of said hub (4) via ultra-sonic atomisation.

## Patentansprüche

1. Nadelbaugruppe, die anpassbar mit einer Spritze (16) genutzt werden kann, die umfasst:
eine Nabe (4) und
eine Nadel (6), die aus der Nabe herausragt;
**gekennzeichnet durch:**
einen flüssigen Gerinnungshemmer, der auf der inneren Fläche der Nadel (6) verteilt und getrocknet wird, um eine Schicht kristallisierten Gerinnungshemmers auf der inneren Fläche der Nadel (6) zu bilden, bevor die Nadel (6) genutzt wird, um in die Vene eines Patienten gestochen zu werden, wobei die Schicht des Gerinnungshemmers verhindert, dass das von dem Patienten abgezapfte Blut in der Nadel (6) gerinnt.

2. Nadelbaugruppe nach Anspruch 1, wobei der Gerinnungshemmer eine Heparin-Substanz umfasst.

3. Nadelbaugruppe nach Anspruch 2, in der die Heparin-Substanz ein Lithium-Heparin-Pulver, vermischt mit deionisiertem Wasser, umfasst.

4. Nadelbaugruppe nach Anspruch 2 oder Anspruch 3, in der die Heparin-Substanz ein Ammonium-Heparin, Zink-Heparin, Kalzium-Heparin, Heparin mit niedrigem Molekulargewicht, Sodium-Heparin, Benzalkonium-Heparin, Dermatom-Heparin, Heparin-Teile und verschiedene Arten von Heparin-Peptidoglycan umfasst.

5. Nadelbaugruppe nach einem der vorangehenden Ansprüche, das zudem umfasst:
eine Hülle (26), die während der Benutzung flexibel mit einer Spritze (16) verbunden ist, um die Nadel (6) fest zu umhüllen, nachdem Nadel (6) benutzt wurde.

6. Nadelbaugruppe nach einem der vorangehenden Ansprüche, wobei der flüssige Gerinnungshemmer zumindest auf einem Teil der inneren Fläche der Nabe (4) verteilt und darauf getrocknet wird, um eine trockene Schicht Gerinnungshemmer darauf zu bilden.

7. Nadel- und Spritzenbaugruppe, die eine Nadelbaugruppe nach einem der vorangehenden Ansprüche umfasst und
einen Filter (20), der anpassungsfähig ist, Luft durchzulassen, aber den Austritt von Flüssigkeit zu blockieren, und der in der Spritze (16) eingebaut ist, um zu ermöglichen, dass die Luftblasen in dem vom Patienten entnommenen und in der Spritze (16) gespeicherten Blut durch den Filter (20) entweichen können.

8. Verfahren zur Herstellung einer Vorrichtung zur Verhinderung der Gerinnung, um gerinnungsfähige Flüssigkeit von einem Patienten zu entnehmen, welche die Schritte umfasst:
a) Befestigung einer Nadel (6) an einer Nabe (4); und
b) Sprühen eines flüssigen Gerinnungshemmers in die Nadel (6), um eine Schicht kristallisierten Gerinnungshemmers auf der inneren Fläche der Nadel (6) zu bilden, bevor die Nadel benutzt wird, um in die Vene eines Patienten zu stechen, wobei die Schicht des Gerinnungshemmers verhindert, dass das von dem Patienten entnommene Blut in der Nadel gerinnt.

9. Verfahren nach Anspruch 8, wobei der Schritt b) zusätzlich den Schritt des Sprühens des Gerinnungshemmers auf zumindest einen Teil der inneren Fläche der Nabe (4) umfasst.

10. Verfahren nach Anspruch 8 oder Anspruch 9, das zudem die Schritte umfasst:
Verbindung der Nadel (6) mit einer Spritze (16); und
flexible Anbringung einer Hülle (26) an der Spritze (16), wobei die Hülle (26) drehbar ist, um die Nadel (6) fest zu umhüllen.

11. Verfahren nach einem der Ansprüche 8 bis 10, das zudem die Schritte umfasst
Verbindung der Nabe (4) mit einer Spritze (16); und
Einbau eines Filters (20), der anpassungsfähig ist, um Luftdurchlass zu erlauben, aber den Austritt von Flüssigkeit in die Spritze zu blockieren, um zu ermöglichen, dass Luftblasen aus der einem Patienten entnommen Flüssigkeit dort entweichen können.

12. Verfahren nach einem der Ansprüche 8 bis 10, die zudem die Schritte umfassen:
Trocknung des Gerinnungshemmers, nachdem er auf die innere Fläche der Nadel (6) gesprüht wurde.

13. Verfahren nach einem der Ansprüche 8 bis 11, wobei der Gerinnungshemmer eine Heparin-Substanz umfasst und worin der Schritt b) zusätzlich den Schritt umfasst
Sprühen der Heparin-Substanz auf die innere Fläche der Nadel (6) durch Sprühen mit Ultraschall.

14. Verfahren nach einem der Ansprüche 8 bis 12, das zusätzlich den Schritt umfasst:
Abgabe des Gerinnungshemmers zum Besprühen der inneren Fläche der Nadel (6) in einer Menge und/oder Konzentration, die abhängig ist von der Länge der Nadel (6).

15. Verfahren nach einem der Ansprüche 8 bis 13, worin der Schritt b) zusätzlich das Besprühen der inneren Fläche der Nadel (6) mit einem Lithium-Heparin-Pulver, das mit deionisiertem Wasser vermischt ist, umfasst.

16. Verfahren nach einem der Ansprüche 8 bis 14, wobei der Schritt b) zusätzlich das Besprühen der inneren Fläche der Nadel (6) mit einem von Ammonium-Heparin, Zink-Heparin, Kalzium-Heparin, Heparin mit niedrigem Molekulargewicht, Sodium-Heparin, Benzalkonium-Heparin, Dermatom-Heparin, Heparin-Teilen und verschiedenen Arten von Heparin-Peptidoglycan umfasst.

17. Verfahren nach einem der Ansprüche 8 bis 15, wobei es den zusätzlichen Schritt umfasst, die Menge und/oder Konzentration des Gerinnungshemmers, mit dem die innere Fläche der Nadel (6) besprüht wird, zumindest entsprechend der Länge der Nadel (6) und der gewünschten Konzentration des Gerinnungshemmers in der Nadel (6) zu variieren.

18. Verfahren nach einem der Ansprüche 9 bis 16, wobei der Gerinnungshemmer mit Ultraschall-Sprühung auf die innere Fläche der Nadel (6) gesprüht wird.

19. Verfahren nach einem der Ansprüche 8 bis 17, das den zusätzlichen Schritt umfasst, bei dem Gerinnungshemmer auf zumindest einen Teil der inneren Fläche der Nabe (4) mit Ultraschall-Sprühung gesprüht wird.

## Revendications

1. Ensemble d'aiguille adaptable pouvant être utilisé avec une seringue (16), comprenant :
un cylindre (4) ; et
une aiguille (6) s'étendant à partir dudit cylindre ;
**caractérisé par** : un anticoagulant liquide distribué et séché sur la surface intérieure de ladite aiguille (6) pour former une couche d'anticoagulant cristallisé sur celle-ci, sur la surface intérieure de ladite aiguille (6) avant que ladite aiguille (6) ne soit utilisée pour piquer la veine d'un patient, de telle sorte que ladite couche d'anticoagulant empêche le sang prélevé du patient de coaguler dans ladite aiguille (6).

2. Ensemble d'aiguille de la revendication 1, dans lequel ledit coagulant comprend une substance d'héparine.

3. Ensemble d'aiguille selon la revendication 2, dans lequel ladite substance d'héparine comprend de la poudre d'héparine lithium mélangée avec de l'eau désionisée.

4. Ensemble d'aiguille selon la revendication 2 ou 3, dans lequel ladite substance d'héparine comprend l'un quelconque parmi l'héparine d'ammonium, l'héparine de zinc, l'héparine de calcium, l'héparine à faible poids moléculaire, l'héparine de sodium, l'héparine de benzalkonium, l'héparine de dermatome, des fragments d'héparine et différentes formes de peptidoglycanes d'héparine.

5. Ensemble d'aiguille selon l'une quelconque des revendications précédentes, comprenant de plus :
une gaine (26) accouplée de façon souple, dans l'utilisation, à une seringue (16) pour envelopper solidement ladite aiguille (6) après que ladite aiguille (6) ait été utilisée.

6. Ensemble d'aiguille selon l'une quelconque des revendications précédentes, dans lequel l'anticoagulant liquide est distribué et séché sur au moins une portion de la surface intérieure dudit cylindre (4) pour former sur celui-ci une couche d'anticoagulant sèche.

7. Ensemble d'aiguille et de seringue comprenant un ensemble d'aiguille selon l'une quelconque des revendications précédentes, et
un filtre (20) pouvant être adapté pour laisser passer l'air mais bloquer le passage de liquide, fixé sur ladite seringue (16) pour permettre à toute bulle d'air dans ledit sang recueilli à partir du patient et étant stocké dans ladite seringue (16) de traverser ledit filtre (20).

8. Procédé de fabrication d'un dispositif anticoagulant pour prélever un fluide coagulant à partir d'un patient, comprenant les étapes consistant à :
a) fixer une aiguille (6) sur un cylindre (4) ; et
b) atomiser un anticoagulant liquide dans ladite aiguille (6) pour former une couche d'anticoagulant cristallisé sur la surface intérieure de ladite aiguille (6) avant que ladite aiguille ne soit utilisée pour piquer la veine d'un patient, de sorte que ladite couche d'anticoagulant empêche le sang prélevé du patient de coaguler dans ladite aiguille.

9. Procédé selon la revendication 8, dans lequel ladite étape b) comprend de plus l'étape d'atomisation dudit anticoagulant sur au moins une portion de la surface intérieure dudit cylindre (4).

10. Procédé selon la revendication 8 ou 9, comprenant de plus les étapes consistant à :
adapter ladite aiguille (6) sur une seringue (16) ; et
accoupler de façon souple une gaine (26) sur ladite seringue (16), ladite gaine (26) étant pivotante pour envelopper solidement ladite aiguille (6).

11. Procédé selon l'une quelconque des revendications 8 à 10, comprenant de plus les étapes consistant à :
adapter ledit cylindre (4) sur une seringue (16) ; et
ajuster un filtre (20) adaptable pour permettre à l'air de passer mais bloquer le passage du fluide vers ladite seringue pour permettre à toute bulle d'air dans ledit fluide prélevé d'un patient de la traverser.

12. Procédé selon l'une quelconque des revendications 8 à 10, comprenant de plus les étapes consistant à :
sécher ledit anticoagulant après avoir été atomisé sur la surface intérieure de ladite aiguille (6).

13. Procédé selon l'une quelconque des revendications 8 à 11, dans lequel ledit anticoagulant comprend une substance d'héparine, et dans lequel ladite étape b) comprend de plus l'étape consistant à :
atomiser ladite substance d'héparine sur la surface intérieure de ladite aiguille (6) par l'intermédiaire d'atomisation ultrasonore.

14. Procédé selon l'une quelconque des revendications 8 à 12, comprenant de plus l'étape consistant à :
distribuer ledit anticoagulant pour atomiser la surface intérieure de ladite aiguille (6) dans un volume et/ou une concentration en fonction de la longueur de ladite aiguille (6).

15. Procédé selon l'une quelconque des revendications 8 à 13, dans lequel ladite étape b) comprend de plus l'atomisation de la surface intérieure de ladite aiguille (6) avec une poudre d'héparine de lithium mélangée avec de l'eau désionisée.

16. Procédé selon l'une quelconque des revendications 8 à 14, dans lequel ladite étape b) comprend de plus l'atomisation de la surface intérieure de ladite aiguille (6) avec l'un quelconque parmi l'héparine d'ammonium, l'héparine de zinc, l'héparine de calcium, l'héparine à faible poids moléculaire, l'héparine de sodium, l'héparine de benzalkonium, l'héparine de dermatome, des fragments d'héparine et différentes formes de peptidoglycanes d'héparine.

17. Procédé selon l'une quelconque des revendications 8 à 15, comprenant l'étape supplémentaire consistant à faire varier le volume et/ou la concentration dudit anticoagulant atomisé sur la surface intérieure de ladite aiguille (6) en fonction d'au moins la longueur de ladite aiguille (6) et de la concentration d'anticoagulant souhaitée dans ladite aiguille (6).

18. Procédé selon l'une quelconque des revendications 9 à 16, dans lequel ledit anticoagulant est atomisé sur la surface intérieure de ladite aiguille (6) par l'intermédiaire d'atomisation ultrasonore.

19. Procédé selon l'une quelconque des revendications 8 à 17, comprenant l'étape supplémentaire d'atomisation de l'anticoagulant sur au moins une portion de la surface intérieure dudit cylindre (4) par l'intermédiaire d'atomisation ultrasonore.
